(19) Europäisches Patentamt

European Patent Office

Office européen des brevets

(11) Veröffentlichungsnummer: **0 621 261 A1**

## (12) EUROPÄISCHE PATENTANMELDUNG

(21) Anmeldenummer: **94102872.2**

(22) Anmeldetag: **25.02.94**

(51) Int. Cl.5: **C07C 213/10, C07C 215/12**

(30) Priorität: **22.04.93 DE 4313135**

(43) Veröffentlichungstag der Anmeldung:
**26.10.94 Patentblatt 94/43**

(84) Benannte Vertragsstaaten:
**DE ES FR GB IT NL**

(71) Anmelder: **Krupp Koppers GmbH**
**Altendorfer Strasse 120**
**D-45143 Essen (DE)**

(72) Erfinder: **Pohl, Werner**
**Laurentiusweg 137**
**D-45276 Essen (DE)**
Erfinder: **Menzel, Johannes**
**Grillostrasse 49**
**D-46045 Oberhausen (DE)**

(54) **Verfahren zur Reinigung einer wässrigen Methyldiethanolaminlösung.**

㊳ Bei diesem Verfahren wird in der zwischen dem Absorber und dem Desorber der Schwefelwasserstoffwäsche umlaufenden MDEA-Lösung der Formiatgehalt gemessen, und bei Erreichen eines Grenzwertes von 0,5 bis 30 g/l wird ein Teilstrom der MDEA-Lösung abgezogen und zwecks Formiat-Entfernung über einen Ionenaustauscher geleitet. Anschließend wird der Teilstrom in den Lösungskreislauf zurückgeführt. Die Regeneration des beladenen Ionenaustauschers erfolgt mit einer Alkalihydroxidlösung, die danach in den Ammoniakabtreiber eingeleitet wird.

EP 0 621 261 A1

Die Erfindung betrifft ein Verfahren zur Reinigung einer wäßrigen Methyldiethanolaminlösung, die zur absorptiven Entfernung von Schwefelwasserstoff aus Gasen genutzt wird, die durch Vergasung von kohlenstoffhaltigem Material, insbesondere von feinkörniger bis staubförmiger Kohle, gewonnen werden.

Die Gase der vorstehend genannten Art enthalten als Verunreinigungen insbesondere Ammoniak ($NH_3$), Cyanwasserstoff (HCN), Schwefelwasserstoff ($H_2S$) und Kohlenoxisulfid (COS). Da diese Verunreinigungen bei der weiteren Verwendung bzw. Verarbeitung der Gase stören, müssen sie zuvor in geeigneter Weise aus dem Gas entfernt werden. Hierfür sind bereits verschiedene Verfahren bekannt. In der Regel wird dabei durch eine Kreislaufwasserwäsche das im Gas enthaltene Ammoniak nahezu vollständig und der Cyanwasserstoff sowie der Schwefelwasserstoff teilweise aus dem Gas entfernt. Das Ammoniak, der Cyanwasserstoff und der Schwefelwasserstoff werden anschließend in getrennten Abtreibern aus dem Kreislaufwasser abgetrieben. Für die Entfernung des noch im Gas vorhandenen Schwefelwasserstoffes hat sich die Verwendung einer wäßrigen Methyldiethanolaminlösung, nachfolgend MDEA-Lösung genannt, als besonders geeignet erwiesen. Der Schwefelwasserstoff wird hierbei absorptiv an der MDEA-Lösung gebunden und kann anschließend aus der beladenen Lösung abgetrieben werden. Bei der praktischen Anwendung dieser Arbeitsweise hat sich jedoch gezeigt, daß es infolge des im Gas noch vorhandenen HCN-Gehaltes im Laufe der Zeit zu einer Bildung und Anreicherung von Formiat in der MDEA-Lösung kommt. Das Formiat wird dabei durch Hydrolyse des HCN zu Ameisensäure und anschließende Umsetzung mit dem Amin gebildet. Durch den Formiatgehalt wird aber die Absorptionsfähigkeit der MDEA-Lösung für Schwefelwasserstoff beeinträchtigt, und es besteht daher die Notwendigkeit, das Formiat in geeigneter Weise aus der MDEA-Lösung zu entfernen.

Der Erfindung liegt deshalb die Aufgabe zugrunde, hierfür ein möglichst kostengünstiges Verfahren zu schaffen, das in möglichst sinnvoller Weise mit dem Gasreinigungsprozeß verknüpft ist und die Zufuhr von frischer MDEA-Lösung zur Aufrechterhaltung der MDEA-Konzentration vermeidet.

Das der Lösung dieser Aufgabe dienende Verfahren ist erfindungsgemäß gekennzeichnet durch die Anwendung folgender Verfahrensmerkmale:

a) In der zwischen dem Absorber und dem Desorber der Schwefelwasserstoffwäsche im Kreislauf geführten Methyldiethanolaminlösung wird der Formiatgehalt gemessen, und bei Erreichen eines Wertes von 0,5 bis 30 g/l wird kontinuierlich ein Teilstrom aus dem Lösungskreislauf abgezogen,

b) der abgezogene Teilstrom wird über einen Ionenaustauscher geleitet, an dem das Formiat gebunden wird,

c) anschließend wird der Teilstrom in den Lösungskreislauf zurückgeführt,

d) der Ionenaustauscher wird mit entmineralisiertem Wasser gespült, das anschließend ebenfalls in den Lösungskreislauf eingespeist wird, und

e) der Ionenaustauscher wird mit einer Alkalihydroxidlösung regeneriert, wobei die dabei anfallende formiathaltige Lauge in den Ammoniakabtreiber der Gasbehandlung eingeleitet wird.

Für die Durchführung des erfindungsgemäßen Verfahrens ist hierbei insbesondere ein basischer, vorzugsweise ein stark basischer, Anionenaustauscher geeignet.

Damit die Reinigung des aus dem Lösungskreislauf abgezogenen Teilstromes kontinuierlich erfolgen kann, ist es zweckmäßig, zwei parallelgeschaltete Ionenaustauschersäulen vorzusehen, die jeweils abwechselnd beladen und regeneriert werden.

Der abgezogene Teilstrom ist abhängig vom HCN-Gehalt des zu reinigenden Gases und beträgt zwischen 0,05 und 5 Vol.-% der im Lösungskreislauf geführten MDEA-Lösung.

Da die bei der Regenerierung des Ionenaustauschers anfallende formiathaltige Lauge erfindungsgemäß in den Ammoniakabtreiber der Gasbehandlung eingeleitet wird, ist für sie keine spzielle und aufwenige Entsorgung erforderlich.

Dieses Vorgehen ist auch deshalb besonders zweckmäßig, da für die Abtreibung des Ammoniaks aus dem Abwasser der Kreislaufwasserwäsche ohnehin eine Lauge, vorzugsweise Natronlauge, dem Abwasser zugesetzt werden muß. Das gebildete Formiat kann dabei ohne Schwierigkeiten bei der biologischen Feinreinigung des Abwassers abgebaut werden.

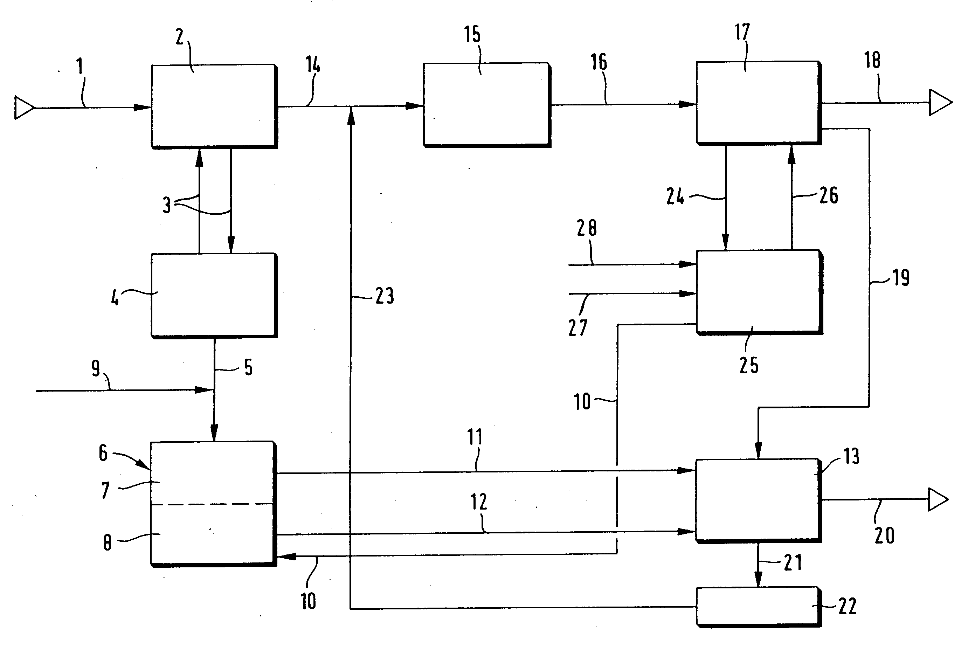

Die Verknüpfung des erfindungsgemäßen Verfahrens mit dem Gasreinigungsprozeß soll nachfolgend an Hand des in der Abbildung dargestellten Fließbildes erläutert werden. Das Fließbild betrifft dabei als Ausführungsbeispiel die Gasreinigung für einen Anwendungsfall, bei dem das gereinigte Gas anschließend in der Brennkammer der Gasturbine eines Kraftwerkes verbrannt werden soll.

Hierbei wird das von der Vergasungsanlage kommende Rohgas über die Leitung 1 in die Kreislaufwasserwäsche 2 eingeleitet. Hier werden bei einem Druck zwischen 15 und 25 bar sowie einer Temperatur zwischen 110 und 150 °C neben dem Staub gleichzeitig das im Gas vorhandene Ammoniak nahezu vollständigund der Cynwasserstoff sowie der Schwefelwasserstoff teilweise ausgewaschen.

Der für die Durchführung der Kreislaufwasserwäsche 2 erforderliche Druck ist normalerweise bereits vorgegeben, da die vorgeschaltete Vergasung unter Druck durchgeführt wird. Durch die Druckanwendung gelingt es, die für die Gasbehandlung erforderlichen Apparate entsprechend klein zu halten. Die erforderliche Temperatur kann normalerweise bei der im Anschluß an die Vergasung erfolgende Kühlung des Rohgases eingestellt werden. Für die Durchführung der Kreislaufwasserwäsche 2 können Gaswascher üblicher Bauart, die mit Einbauten versehen sein können, eingesetzt werden. Um das Waschwasser von dem mitgeführten Staub, der bei der Kreislaufwasserwäsche 2 aus dem Gas entfernt wird, zu befreien, wird das Waschwasser im Kreislauf über die Feststoffabscheidung 4 geführt, was im Fließbild durch den Doppelpfeil 3 angedeutet wird. Hierbei wird in der Feststoffabscheidung 4 der mitgeführte Staub in an sich bekannter Weise, z.B. durch Filtration und/oder Sedimentation, vom Waschwasser abgetrennt. Anschließend gelangt das Waschwasser in die Kreislaufwasserwäsche 2 zurück.

Um eine unerwünschte Anreicherung der gelösten Schadstoffe im Waschwasser zu vermeiden, wird jeweils ein kleiner Teilstrom desselben im Anschluß an die Feststoffabscheidung 4 abgezogen und über die Leitung 5 der zweistufigen Strippung 6 zugeführt. Die Menge des über die Leitung 5 abgezogenen Teilstromes ist dabei vom Schadstoffgehalt, insbesondere vom Chloridgehalt, des für die Vergasung eingesetzten Brennstoffes abhängig. Soweit es erforderlich ist, wird die aus dem Kreislauf abgezogene Waschwassermenge durch Frischwasser ersetzt. In der zweistufigen Strippung 6 ist eine Hintereinanderschaltung eines im sauren Milieu arbeitenden Abtreibers 7 und eines im basischen Milieu arbeitenden Abtreibers 8 vorgesehen. Zunächst werden in dem im sauren Milieu arbeitenden Abtreiber 7 die sauren Komponenten aus dem Waschwasser abgetrieben. Durch die Zugabe von Säure werden diese Komponenten gemäß den Reaktionsgleichungen

$$CN^- + H^+ \rightarrow HCN$$
$$HS^- + H^+ \rightarrow H_2S$$

in die molekulare Form überführt und anschließend durch Temperaturerhöhung abgetrieben. Die hierfür erforderliche Säure, wie z.B. Salzsäure oder Schwefelsäure wird über die Leitung 9 dem Teilstrom in der Leitung 5 am Stripperzulauf zudosiert.

Anschließend wird das aus dem Abtreiber 7 ablaufende Waschwasser in den Abtreiber 8 überführt. Hier werden durch Zugabe von Lauge die im Waschwasser enthaltenen Ammoniumionen gemäß der Reaktionsgleichung

$$NH_4^+ + OH^- \rightarrow NH_3 + H_2O$$

in molekulares Ammoniak überführt, das ebenfalls durch Temperaturerhöhung aus dem Waschwasser abgetrieben wird. Erfindungsgemäß wird hierfür die bei der Regenerierung des Ionenaustauschers 25 anfallende formiathaltige Lauge über die Leitung 10 in den Abtreiber 8 eingeleitet. Falls die auf diese Weise zugeführte Laugenmenge nicht ausreicht, kann zusätzlich weitere freie Lauge, wie z.B. Natronlauge, zudosiert werden.

Im Anschluß an den Abtreiber 8 kann das entsprechend behandelte Waschwasser entweder zur Kreislaufwasserwäsche 2 zurückgeführt werden, oder es wird aus dem Verfahren ausgeschleust und einer biologische Abwasserbehandlungseinrichtung zugeführt. Beide Möglichkeiten sind im Fließbild nicht dargestellt. Die zweistufige Strippung 6 wird in Abtreiberkolonnen üblicher Bauart durchgeführt, bei denen die für den Abtrieb der Schadstoffe aus dem Waschwasser erforderliche Temperaturerhöhung durch einen Sumpfumlaufkocher bewirkt wird. Die aus dem Waschwasser abgetriebenen Schadstoffe, d.h. HCN, $H_2S$ und $NH_3$, werden über die Leitungen 11 und 12 der Claus-Anlage 13 zugeführt. In Abweichung von der im Fließbild vorgesehenen getrennten Zufuhr können die aus den beiden Abtreibern 7 und 8 austretenden Schadstoffströme auch vereinigt und über eine gemeinsame Leitung zur Claus-Anlage 13 geführt werden.

Das aus der Kreislaufwasserwäsche 2 austretende entstaubte Gas wird währenddessen über die Leitung 14 der Kohlenoxisulfid-Entfernung 15 zugeführt. Hier wird das im Gas vorhandene Kohlenoxisulfid, das mit Wasser oder anderen üblichen Lösungsmitteln nur schwer auszuwaschen ist, durch katalytische Hydrolyse in Schwefelwasserstoff überführt. Dabei reagiert das Kohlenoxisulfid in der Gasphase mit Wasserdampf gemäß folgender Reaktionsgleichung:

$$COS + H_2O \rightarrow H_2S + CO_2$$

Der für diese Umsetzung verwendete Katalysator enthält Aluminiumoxid als aktive Komponente. Anschließend gelangt das Gas über die Leitung 16 zur Schwefelwasserstoffwäsche 17, in der der im Gas vorhandene Schwefelwasserstoff absorptiv mit der MDEA-Lösung ausgewaschen wird Dabei kann der im Gas vorhandene Schwefelwasserstoff bis auf einen Restgehalt von ca. 7 ppm entfernt werden, während die Coabsorption der übrigen Gaskomponenten nur gering ist. Im Anschluß an die Schwefelwasserstoffwäsche 17 besitzt das behandelte Gas eine ausreichende Reinheit und kann deshalb über die Leitung 18 der Brennkammer der Gasturbine zugeführt werden.

Die Schwefelwasserstoffwäsche 17 besteht aus einer Absorptions- und einer Desorptionskolonne. In letzterer wird die beladene MDEA-Lösung durch Abtreiben des aufgenommenen Schwefelwasserstoffes regeneriert. Dabei fällt ein Gasstrom mit einer hohen $H_2S$-Konzentration an, der über die Leitung 19 zur Claus-Anlage 13 gelangt. Hier werden die Gasströme aus den Leitungen 11, 12 und 19 nach einem modifizierten Claus-Prozeß, der auch die katalytische Zersetzung der über die Leitung 11 und 12 zugeführten Stickstoffverbindungen ($NH_3$ und HCN) gestattet, weiterbehandelt.

Der gewonnene Elementarschwefel wird über die Leitung 20 in flüssiger Form aus der Claus-Anlage 13 abgezogen und kann seiner weiteren Verwendung zugeführt werden. In der Claus-Anlage 13 fällt immer ein sogenanntes Claus-Restgas an, das neben nicht kondensiertem Elementarschwefel nicht umgesetztes Schwefeldioxid enthält. Da dieses Claus-Restgas wegen seines Schadstoffgehaltes nicht ohne weiteres in die Atmosphäre abgelassen werden kann, muß es einer Nachbehandlung unterworfen werden. Das aus der Claus-Anlage 13 austretende Claus-Restgas wird deshalb über die Leitung 21 der Nachbehandlung 22 zugeführt. Bei der im Fließbild dargestellten Ausführungsform des Verfahrens erfolgt die Nachbehandlung durch katalytische Hydrierung.

Das hierbei anfallende $H_2S$-haltige Gas wird über die Leitung 23 zurückgeführt und nach entsprechender Verdichtung dem Gasstrom in Leitung 14 vor dessen Eintritt in die Kohlenoxisulfid-Entfernung 15 zugemischt.

Aus der in der Schwefelwasserstoffwäsche 17 umlaufenden MDEA-Lösung wird erfindungsgemäß über die Leitung 24 ein Teilstrom abgezogen und zwecks Entfernung des gebildeten Formiats aus der Lösung über den Ionenaustauscher 25 geleitet. Anschließend wird die gereinigte MDEA-Lösung über die Leitung 26 in den Lösungskreislauf in der Schwefelwasserstoffwäsche 17 zurückgeführt. Zur Vermeidung von MDEA-Verlusten wird der Ionenaustauscher 25 anschließend mit entmineralisiertem Wasser gespült, das über die Leitung 27 zugeführt wird. Nach Passieren des Ionenaustauschers 25 gelangt das Spülwasser über die Leitung 26 ebenfalls in die Schwefelwasserstoffwäsche 17 und dient dort zum Ausgleich von Wasserverlusten bei der $H_2S$-Desorption. Die für die Regenerierung des Ionenaustauschers 25 erforderliche Kalilauge wird über die Leitung 28 zugeführt. Die bei der Regenerierung anfallende formathaltige Lauge wird, wie bereits weiter oben beschrieben wurde, über die Leitung 10 abgezogen und in den Abtreiber 8 eingeleitet.

**Patentansprüche**

1. Verfahren zur Reinigung einer wäßrigen Methyldiethanolaminlösung, die zur absorptiven Entfernung von Schwefelwasserstoff aus Gasen genutzt wird, die durch Vergasung von kohlenstoffhaltigem Material, insbesondere von feinkörniger bis staubförmiger Kohle, gewonnen werden, **gekennzeichnet durch** folgende Verfahrensmerkmale:

   a) In der zwischen dem Absorber und dem Desorber der Schwefelwasserstoffwäsche im Kreislauf geführten Methyldiethanolaminlösung wird der Formiatgehalt gemessen, und bei Erreichen es Wertes von 0,5 bis 30 g/l wird kontinuierlich ein Teilstrom aus dem Lösungskreislauf abgezogen,

   b) der abgezogene Teilstrom wird über einen Ionenaustauscher geleitet, an dem das Formiat gebunden wird,

   c) anschließend wird der Teilstrom in den Lösungskreislauf zurückgeführt,

   d) der Ionenaustauscher wird mit entmineralisiertem Wasser gespült, das anschließend ebenfalls in den Lösungskreislauf eingespeist wird,

   und

   e) der Ionenaustauscher wird mit einer Alkalihydroxidlösung regeneriert, wobei die dabei anfallende formiathaltige Lauge in den Ammoniakabtreiber der Gasbehandlung eingeleitet wird.

2. Verfahren nach Anspruch 1 , **dadurch gekennzeichnet,** daß als Ionenaustauscher ein basischer, vorzugsweise stark basischer, Anionenaustauscher zur Anwendung gelangt.

3. Verfahren nach den Ansprüchen 1 und 2, **dadurch gekennzeichnet,** daß zwei parallelgeschaltete Ionenaustauscher vorgesehen sind, die jeweils abwechselnd beladen und regeneriert werden.

4. Verfahren nach den Ansprüchen 1 bis 3, **dadurch gekennzeichnet,** daß zwischen 0,05 und 5 Vol.-% der im Lösungskreislauf geführten Methyldiethanolaminlösung als Teilstrom abgezogen werden.

5. Verfahren nach den Ansprüchen 1 bis 4, **dadurch gekennzeichnet,** daß für die Regeneration des Ionenaustauschers Natronlauge verwendet wird.

EP 0 621 261 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | US-A-4 970 344 (ALFRED E. KELLER) <br> * das ganze Dokument * <br> --- | 1-3,5 | C07C213/10 <br> C07C215/12 |
| X | US-A-2 797 188 (FRANK CARTER TAYLOR ET AL.) <br> * das ganze Dokument * <br> --- | 1-3,5 | |
| X | US-A-4 795 565 (TSOUNG Y. YAN) <br> * das ganze Dokument * <br> --- | 1-3,5 | |
| A | EP-A-0 286 143 (UNION CARBIDE CORPORATION) <br> * Zusammenfassung * <br> ----- | 1 | |

RECHERCHIERTE SACHGEBIETE (Int.Cl.5)

C07C

Der vorliegende Recherchenbericht wurde für alle Patentansprüche erstellt

| Recherchenort | Abschlußdatum der Recherche | Prüfer |
|---|---|---|
| BERLIN | 9. Mai 1994 | Rufet, J |